# EUROPEAN PATENT APPLICATION

(11) **EP 4 052 698 A1**
(43) Date of publication of application: **07.09.2022**
(21) Application number: 22160169.3
(22) Date of filing: 04.03.2022
(51) Int. Cl.: A61K 9/20, A61K 31/4196

(54) **FILM COATED TABLET COMPRISING DEFERASIROX**

(30) Priority: 05.03.2021 TR 202104272
(71) Applicant: SANOVEL ILAC SANAYI VE TICARET A.S., Istanbul 34460 (TR)
(72) Inventor: EMEN, Seyhmus, Istanbul (TR); ERDOGAN, Akif, Istanbul (TR); BILGEHAN ATAK, Fadime, Istanbul (TR); SUNEL, Fatih, Istanbul (TR)
(74) Representative: Sevinç, Erkan

(57) **Abstract**

The present invention relates to a film coated tablet comprising deferasirox and at least one pharmaceutically acceptable excipient. Furthermore, the present invention discloses a process for the preparation of a film coated tablet formulation comprising deferasirox. The process is simple, rapid, cost effective, time-saving and industrially convenient process and is created to eliminate the disadvantages of active ingredient.

## Description

### Field of the Invention

The present invention relates to a film coated tablet comprising deferasirox and at least one pharmaceutically acceptable excipient. Furthermore, the present invention discloses a process for the preparation of a film coated tablet formulation comprising deferasirox. The process is simple, rapid, cost effective, time-saving and industrially convenient process and is created to eliminate the disadvantages of active ingredient.

### Background of the Invention

Deferasirox is an iron-binding agent used for the treatment of iron overload. Its chemical name is 4-[3,5-bis(2-hydroxyphenyl)[1,2,4]triazole-1-yl]benzoic acid and has the structure shown below in formula I.

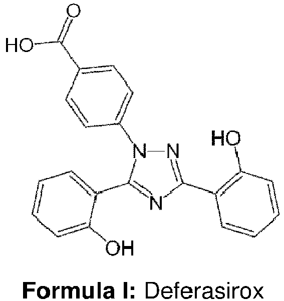

Deferasirox is indicated in the treatment of iron overload for reducing iron-related morbidity and mortality in transfusion-dependent anemia, particularly in mild and severe thalassemia, and in sickle cell anemia. At the same time, deferasirox can be used in the treatment of hemochromatosis. The red blood cells in the body of patients afflicted with mild and severe thalassemia, sickle-cell anemia, and hemochromatosis are exposed to a very rapid destruction and a significant amount of blood transfusion is given to the patients in the need thereof. If the excessive amount of iron following a blood transfusion is not eliminated from the body, it may cause organ failures. Deferasirox is an effective active agent in eliminating excessive iron from the body.

Deferasirox is the first oral drug approved for the treatment of iron overload in the United States. It was approved by FDA (Food and Drug Administration) in November 2005.

Deferasirox, which is commercially-available in water-dispersible forms (EXJADE^{®}) was first disclosed in the patent WO97/49395, wherein it was indicated for the treatment of diseases caused by excessive iron in the body.

It is known that deferasirox is poorly soluble in water. For this reason, the most-frequently encountered difficulty in creating a formulation comprising deferasirox is the solubility thereof. Additionally, the particles obtained in the production of tablets of deferasirox have low flow properties and are prone to adhere to each other because of the low density and the electrostatic characteristics of deferasirox.

WO 2009/135948 A2 patent application discloses a method for producing a tablet comprising (a) a first active ingredient in an amount 50-90% w/w and (b) one or more excipients including at least a binder in an amount 10-50% w/w.

WO 2009/016359 A1 patent application discloses pharmaceutical formulations comprising; a therapeutically effective amount of a stable anhydrous form of deferasirox; and a pharmaceutically acceptable excipient, wherein said deferasirox is substantially free of its acid addition salts. Further the application is directed to prepare said pharmaceutical formulations and to methods of using the formulations to treat conditions in a subject in need thereof.

In order to fulfill all the requirements described above, a special drug formulation should be adapted particularly by a careful selection of the excipients to be used. Therefore, a film coated tablet comprising deferasirox are desired, which would not lead to losses in the active agents or excipients during production, i.e. would have a high production yield, have good flow properties, and would be simply compressed into tablets. Other advantages and embodiments of the present invention will be clarified in the following description.

### Detailed Description of the Invention

The main object of the present invention is to eliminate problems and bringing additional advantages to the relevant prior art.

The more clearly main object of the present invention is to provides a film coated tablet comprising deferasirox having desired dissolution profile and desired stability and to prevent sticking of active agents.

Another object of the present invention is to provide a tablet having improved content uniformity and flowability.

Another object of the present invention is to provides a process for a film coated tablet comprising deferasirox. The process is a simple, rapid, cost effective, time-saving and industrially convenient method.

According to one embodiment of the present invention, a film coated tablet comprises deferasirox and at least one pharmaceutically acceptable excipient wherein the weight ratio of at least one binder to at least one lubricant/glidant is between 1.0 and 8.0, preferably the ratio is between 1.0 and 4.0. the ratio helps to prevent sticking and improve flowability.

Suitable binders are selected from the group comprising polyvinylpyrrolidone, sodium carboxymethyl cellulose, polyethylene glycol, polyvinyl alcohol, pregelatinized starch, glucose, natural gums, sucrose, sodium alginate, hydroxypropyl methyl cellulose, hydroxypropyl cellulose, carboxy methyl cellulose, methyl cellulose, carrageenan, guar gum, carbomer, polymethacrylates, methacrylate polymers, gelatin, alginate, alginic acid, xanthan gum, hyaluronic acid, pectin, polysaccharides, polyacrylamide, polyoxyethylene-alkyl ether, polydextrose, polyethylene oxide or mixtures thereof.

According to one embodiment of the present invention, the amount of binders is between 0.5% and 5.0% by weight in the total core tablet. Preferably, it is between 1.0% and 4.0% by weight in the total core tablet.

According to one embodiment of the present invention, the binder is polyvinylpyrrolidone.

Suitable lubricants/glidants are selected from the group comprising from sodium stearyl fumarate, colloidal silicon dioxide, magnesium stearate, talc, calcium stearate, zinc stearate, waxes, hydrogenated vegetable oil, , magnesium lauryl sulfate, sodium oleate, sodium acetate, sodium benzoate, stearic acid, fatty acid, fumaric acid, glyceryl palmito sulphate or mixtures thereof.

According to one embodiment of the present invention, the amount of lubricants/glidants is between 0.5% and 6.0% by weight in the total core tablet. Preferably, it is between 0.5% and 4.0% by weight in the total core tablet.

Sodium stearyl fumarate is hydrophilic, so it does not have a negative effect on the solubility of the active substance. It also prevents the mixture from sticking during compressing.

According to one embodiment of the present invention, the lubricants/glidants is sodium stearyl fumarate or colloidal silicon dioxide or mixtures thereof. Using these excipients provides the desired flowability and compressibility.

According to one embodiment of the present invention, the amount of deferasirox is present between 35.0% and 68.0% by weight in the total core tablet, preferably, between 40.0% and 66.0% by weight in the total core tablet.

According to one embodiment of the present invention, the amount of deferasirox is present 42.0% or 65.0% by weight in the total core tablet.

In general terms, excipients provided in a formulation may positively or negatively influence the physicochemical and pharmacokinetic properties, e.g. the solubility, absorption, bioavailability of an active agent. For this reason, the excipients which accompany an active agent have to be selected in a careful and conscious manner while a formulation is developed. The formulations should have no physicochemical incompatibility between the active agents and the excipients.

According to one embodiment of the present invention, the film coated tablet comprises at least one pharmaceutically acceptable excipient which is selected from the group comprising fillers, disintegrants, surfactans or mixtures thereof.

Suitable fillers are selected from the group comprising microcrystalline cellulose, lactose, mannitol, spray-dried mannitol, starch, lactose monohydrate, dextrose, sucrose, fructose, maltose, sorbitol, xylitol, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to one embodiment of the present invention, the amount of fillers is between 13.0% and 65.0% by weight in the total core tablet. Preferably, it is between 20.0% and 60.0% or between 40.0% and 55.0% by weight in the total core tablet.

According to one embodiment of the present invention, the filler is microcrystalline cellulose.

Suitable disintegrants are selected from the group comprising crospovidone, croscarmellose sodium, starch, low-substituted hydroxypropyl cellulose, sodium carboxymethyl cellulose, calcium carboxymethyl cellulose, carboxymethyl cellulose, guar gum, polyacryline potassium, sodium alginate, corn starch, sodium starch glycolate, alginic acid, alginates, or mixtures thereof.

According to one embodiment of the present invention, using suitable disintegrant helps to provide the desired dissolution profile.

According to one embodiment of the present invention, the amount of disintegrants is between 1.0% and 10.0% by weight in the total core tablet. Preferably, it is between 4.0% and 9.0% by weight in the total core tablet.

According to one embodiment of the present invention, the disintegrant is crospovidone.

Suitable surfactants are selected from the group comprising poloxamer, benzalconium chloride, docusate sodium, glyceryl esters, glyceryl monoleate, polyethylene alkyl ethers, polyglyceryl esters, polysorbates, sorbitan esters, sodium lauryl esters, polyoxyetylene esters, polyoxyetylene stearates, sodium stearate, calcium oleate, cetrimonium bromide, hexadecyl pyridinium chloride or mixtures thereof.

According to one embodiment of the present invention, the surfactant is poloxamer.

According to one embodiment of the present invention, the amount of surfactants is between 0.01% and 1.0% by weight in the total core tablet.

According to one embodiment of the present invention, inner phase comprises active agent, at least one filler and at least one disintegrant, binder part comprises at least one binder and at least one surfactant, outer phase comprises at least one filler, at least one disintegrant and at least one lubricants/glidants.

A film coat on the tablet protects from moisture and further contributes to the ease with which it can be swallowed. Suitable coating agent are selected from the group comprising polyvinyl alcohol (PVA), talc, GMCC Type 1 (Glycerol monocaprylocaprate Type I) / Glycerol ester, sodium lauryl sulphate, polymethacrylates, hydroxypropyl methylcellulose, lactose monohydrate, hydroxypropyl cellulose, polyethylene glycol (PEG), talc, polyvinyl alcohol-polyethylene glycol copolymers (Kollicoat^{®} IR), polyvinylprolidone, polyvinylprolidone-vinyl acetate copolymer (PVP-VA), all kinds of Opadry^{®}, pigments, dyes, titanium dioxide, iron oxide or mixtures thereof or mixtures thereof. The amount of the coating agent is between 1.5% and 4.0% by weight.

According to one embodiment of the present invention, the film coated tablet comprises;
Inner phase; Deferasirox, microcrystalline cellulose, crospovidone
Binder part; Polyvinylpyrrolidone and poloxamer
Outer phase; Microcrystalline cellulose, crospovidone, colloidal silicon dioxide, sodium stearyl fumarate.

According to one embodiment of the present invention, a process for the preparation of the film coated tablet further comprises the following steps:
a) Sieving deferasirox, microcrystalline cellulose and crospovidone and mixing,
b) Dissolving polyvinylpyrrolidone and poloxamer in water and obtained a granulation solution,
c) Granulating the mixture at step (a) and the granulation solution at step (b),
d) Drying the wet granules at step (c) at fluid bed and obtained dry granules,
e) Sieving the dry granules,
f) Adding microcrystalline cellulose, crospovidone, colloidal silicon dioxide and mixing,
g) Adding sodium stearyl fumarate and mixing,
h) Compressing to form of tablets,
i) Coating tablets with coating agents.

It has surprisingly been found that a film coated tablet of excellent content uniformity, good flowability and showing improved dissolution can be obtained when a mixture comprising deferasirox and a granulation solution comprising a binder (preferably polyvinylpyrrolidone) and a surfactant (preferably poloxamer) is formulated together in wet granulation process. Wet granulation process efficiently counteracts the segregation of active agent, so it is observed that the desired stability, tablet hardness, content uniformity and compressibility.

So, an easy method is created to eliminate the disadvantages of active ingredient and also the method is simple and cost-effective method. Also, this process helps to provide the desired dissolution profile.

### Example 1: The film coated tablet comprising deferasirox

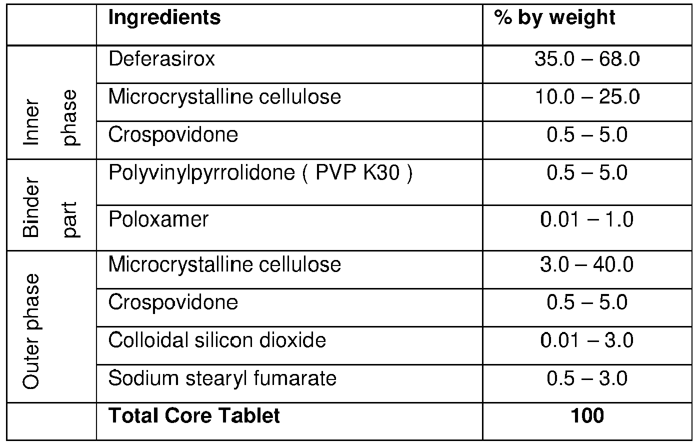

### Example 2: The film coated tablet comprising deferasirox

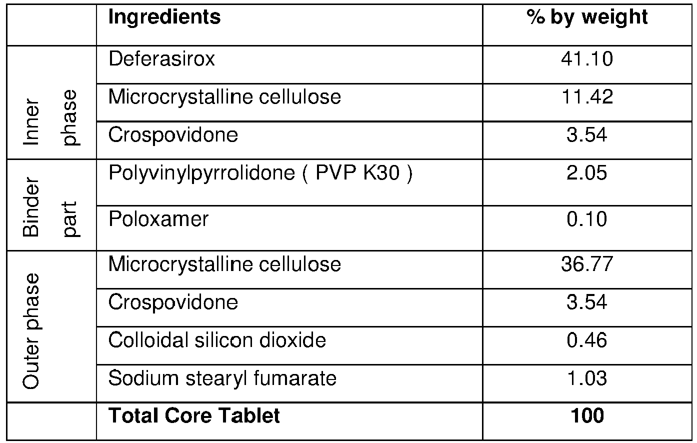

### Example 3: The film coated tablet comprising deferasirox

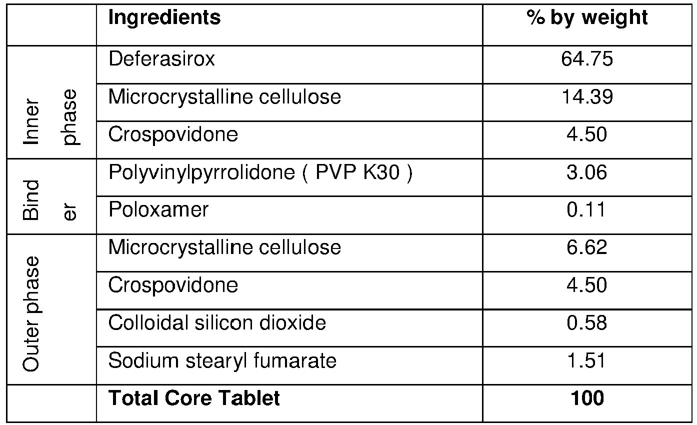

**A process for example 1 or 2 or 3;**
a) Sieving deferasirox, microcrystalline cellulose and crospovidone and mixing,
b) Dissolving polyvinylpyrrolidone and poloxamer in water and obtained a granulation solution,
c) Granulating the mixture at step (a) and the granulation solution at step (b),
d) Drying the wet granules at step (c) at fluid bed and obtained dry granules,
e) Sieving the dry granules,
f) Adding microcrystalline cellulose, crospovidone, colloidal silicon dioxide and mixing,
g) Adding sodium stearyl fumarate and mixing,
h) Compressing to form of tablets,
i) Coating tablets with coating agents.

All sieving process are done through a 1.8 mm sieve.

### Film coating

| **Ingredients** |
|---|
| Hydroxypropyl methylcellulose |
| Titanium dioxide |
| Macrogol/PEG |
| Talc |
| FD&C BLUE |

## Claims

1. A film coated tablet comprising deferasirox and at least one pharmaceutically acceptable excipient wherein the weight ratio of at least one binder to at least one lubricant/glidant is between 1.0 and 8.0, preferably the ratio is between 1.0 and 4.0.

2. The film coated tablet according to claim 1, wherein binders are selected from the group comprising polyvinylpyrrolidone, sodium carboxymethyl cellulose, polyethylene glycol, polyvinyl alcohol, pregelatinized starch, glucose, natural gums, sucrose, sodium alginate, hydroxypropyl methyl cellulose, hydroxypropyl cellulose, carboxy methyl cellulose, methyl cellulose, carrageenan, guar gum, carbomer, polymethacrylates, methacrylate polymers, gelatin, alginate, alginic acid, xanthan gum, hyaluronic acid, pectin, polysaccharides, polyacrylamide, polyoxyethylene-alkyl ether, polydextrose, polyethylene oxide or mixtures thereof.

3. The film coated tablet according to claim 1, wherein the amount of binders is between 0.5% and 5.0% by weight in the total core tablet.

4. The film coated tablet according to claim 2, wherein the binder is polyvinylpyrrolidone.

5. The film coated tablet according to claim 1, wherein lubricants/glidants are selected from the group comprising from sodium stearyl fumarate, colloidal silicon dioxide, magnesium stearate, talc, calcium stearate, zinc stearate, waxes, hydrogenated vegetable oil, , magnesium lauryl sulfate, sodium oleate, sodium acetate, sodium benzoate, stearic acid, fatty acid, fumaric acid, glyceryl palmito sulphate or mixtures thereof.

6. The film coated tablet according to claim 1, wherein the amount of lubricants/glidants is between 0.5% and 6.0% by weight in the total core tablet.

7. The film coated tablet according to claim 5, wherein the lubricants/glidants is sodium stearyl fumarate or colloidal silicon dioxide or mixtures thereof.

8. The film coated tablet according to claim 1, wherein the amount of deferasirox is present between 35.0% and 68.0% by weight in the total core tablet, preferably, between 40.0% and 66.0% by weight in the total core tablet.

9. The film coated tablet according to claim 1, wherein the film coated tablet comprises at least one pharmaceutically acceptable excipient which is selected from the group comprising fillers, disintegrants, surfactans or mixtures thereof.

10. The film coated tablet according to claim 9, wherein fillers are selected from the group comprising microcrystalline cellulose, lactose, mannitol, spray-dried mannitol, starch, lactose monohydrate, dextrose, sucrose, fructose, maltose, sorbitol, xylitol, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

11. The film coated tablet according to claim 9, wherein disintegrants are selected from the group comprising crospovidone, croscarmellose sodium, starch, low-substituted hydroxypropyl cellulose, sodium carboxymethyl cellulose, calcium carboxymethyl cellulose, carboxymethyl cellulose, guar gum, polyacryline potassium, sodium alginate, corn starch, sodium starch glycolate, alginic acid, alginates, or mixtures thereof.

12. The film coated tablet according to claim 9, wherein surfactants are selected from the group comprising poloxamer, benzalconium chloride, docusate sodium, glyceryl esters, glyceryl monoleate, polyethylene alkyl ethers, polyglyceryl esters, polysorbates, sorbitan esters, sodium lauryl esters, polyoxyetylene esters, polyoxyetylene stearates, sodium stearate, calcium oleate, cetrimonium bromide, hexadecyl pyridinium chloride or mixtures thereof.

13. The film coated tablet according to claim 1, wherein the film coated tablet comprises;
- Deferasirox,
- Microcrystalline cellulose,
- Crospovidone,
- Polyvinylpyrrolidone,
- Poloxamer,
- Colloidal silicon dioxide,
- Sodium stearyl fumarate.

14. A process for the preparation of the film coated tablet further comprising the following steps:
a) Sieving deferasirox, microcrystalline cellulose and crospovidone and mixing,
b) Dissolving polyvinylpyrrolidone and poloxamer in water and obtained a granulation solution,
c) Granulating the mixture at step (a) and the granulation solution at step (b),
d) Drying the wet granules at step (c) at fluid bed and obtained dry granules,
e) Sieving the dry granules,
f) Adding microcrystalline cellulose, crospovidone, colloidal silicon dioxide and mixing,
g) Adding sodium stearyl fumarate and mixing,
h) Compressing to form of tablets,
i) Coating tablets with coating agents.
